# EUROPEAN PATENT APPLICATION

(11) **EP 4 813 944 A2**
(43) Date of publication of application: **30.09.2026**
(21) Application number: 26190569.9
(22) Date of filing: 26.07.2024
(51) Int. Cl.: C04B 26/26

(54) **COMPUTER SYSTEM FOR DETERMINING THE COMPOSITION OF THE BITUMINOUS CONGLOMERATE, A PLANT COMPRISING SAID SYSTEM AND CALCULATION METHOD**

(30) Priority: 02.08.2023 IT 202300016479
(62) Document number(s) of the earlier application(s) in accordance with Art. 76 EPC: 24191143.7 / 4 502 070
(71) Applicant: Alma Mater Studiorum Universita di Bologna, 40126 Bologna (IT); MARINI S.p.A., 48011 Alfonsine (Ravenna) (IT)
(72) Inventor: PIOGGIA, Roberto, 48121 RAVENNA (IT); SANGIORGI, Cesare, 40136 BOLOGNA (IT); TATARANNI, Piergiorgio, 40139 BOLOGNA (IT); TARSI, Giulia, 61122 PESARO (IT)
(74) Representative: Milli, Simone

(57) **Abstract**

A computer system (1) for generating mixes for making bituminous macadam starting from basic ingredients,
the system (1) being characterised in that it comprises, in combination:
- a user interface (2), configured to provide displays of information relating to said mixes and allow the entering or modification of one or more of said items of information relating to the mixes;
- a calculation module (3), configured for calculating a percentage of regeneration additive for regenerating the reclaimed bituminous-based material (RAP) on the basis of at least one formula, said formula determining a mathematical relationship between:
- reclaimed bituminous-based material;
- virgin bituminous conglomerate;
- final product obtained from the mix

through a characteristic size of the bitumen.

## Description

This invention relates to a computer system and a method designed to allow the composition of the bituminous mix to be determined in predetermined conditions.

In the sector for the production of bituminous macadam, the prior art teaches the use of a certain percentage of reclaimed bituminous-based material (i.e. RAP), that is to say, deriving from the breaking, for example, of road surfaces or the like; this with the aim of partly recycling the waste material.

A need in this sense is that of being able to process, starting from the percentage of so-called waste mix and other basic ingredients, the optimum mix (that is to say, the composition, in terms of percentages of the ingredients) for producing a bituminous conglomerate of excellent quality.

In this regard, it should be noted that in order to be able to use reclaimed bituminous-based material, it is necessary to use a regenerating regeneration additive in order to allow the production of a final asphalt having predetermined characteristics; the aim of the regenerating additive is to re-activate in an efficient manner the bitumen present in the reclaimed bituminous-based material (RAP) so as to be able to return it as close as possible to the conditions of the so-called virgin bitumen, that is to say, the bitumen never used.

The quantity of regeneration additive which must be used is, currently, determined in an empirical manner by means of empirical tables generally provided by the supplier of the regeneration additive.

In essence, the user of the macadam production plant must use empirical tables provided by the supplier in order to determine the percentage of regenerating additive to be used, for a predetermined quantity of reclaimed bituminous-based material, in order to obtain an end product of the bituminous conglomerate type having predetermined quantities of product.

This situation places serious limitations on practicality and simplicity in determining the quantities of the basic ingredients of mixes based on reclaimed bituminous-based material, and a strongly felt need in the sector in question is therefore to determine the quantity of regenerating regeneration additive to be used for reactivating in an effective manner the bitumen present in the reclaimed bituminous-based material RAP irrespective of the type of regenerating regeneration additive.

It should be noted that, currently, the setting of the various ingredients making up the mix of the bituminous conglomerate is performed by the plant operator substantially by setting percentage values of the various ingredients using information tools; the calculation of the percentage of the so-called waste mix and regenerating regeneration additive must, however, be performed, in an empirical manner, by the operator.

Therefore, there is currently no method which allows the operator to determine simply and quickly all the ingredients of the mix for the virgin bituminous conglomerate, in particular if reclaimed bituminous-based material is used.

The aim of this invention is to satisfy the above-mentioned need and to overcome the above-mentioned limitations of the prior art by providing a system for determining a mix for bituminous macadam and the relative determining method.

In particular, the aim of this invention is to provide a system for determining a mix for bituminous macadam and the relative determining method which are particularly simple and practical in use, allowing the mix for the production of bituminous macadam to be determined in an optimum manner starting from a plurality of parameters.

The technical features of the invention, with reference to the above aims, are clearly described in the claims below and its advantages are more apparent from the detailed description which follows, with reference to the accompanying drawings which illustrate a non-limiting embodiment of the invention by way of an example, and in which:

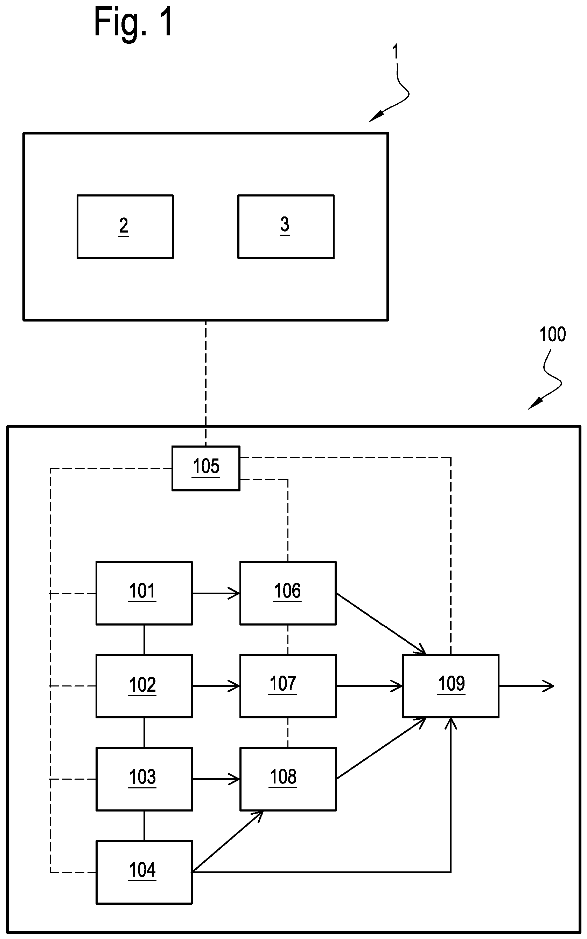

- Figure 1 schematically illustrates a plant for producing bituminous macadam to which the computer system according to the invention is applicable;
- Figures 2 to 3 illustrate graphs of relationships obtained experimentally, by means of experimental tests, by the Applicant, relating to the trend of the content of regenerating additive necessary to obtain a desired depth of penetration;
- Figure 4 illustrate graphs of relationships obtained experimentally, by means of experimental tests, by the Applicant, relating to the trend of the content of regenerating additive necessary to obtain a desired depth of penetration;
- Figures 5 to 9 illustrate respective screens of the user interface of the computer system according to the invention.

According to the invention, the reference numeral 1 denotes a computer system for generating mixes for making bituminous macadam starting from basic ingredients.

The computer system 1 may comprise a processor.

It should be noted that said basic ingredients comprise:
- aggregates (stones or the like);
- virgin bitumen;
- a filler, that is to say, a filling product (for example sand or the like);
- reclaimed bituminous-based material (RAP);
- a regeneration additive for regenerating the reclaimed bituminous-based material (RAP).

It should be noted that the computer system 1 comprises, in combination:
- a user interface 2, configured to provide displays of information relating to said mixes and allow the entering or modification of one or more of said items of information relating to the mixes;
- a calculation module 3, configured for calculating a percentage of regeneration additive for regenerating the reclaimed bituminous-based material (RAP) on the basis of at least one formula, said formula determining a mathematical relationship between:
- reclaimed bituminous-based material;
- virgin bituminous conglomerate;
- final product obtained from the mix
through a characteristic size of the bitumen.

It should be noted that the user interface 2 and the calculation module 3 are software modules, which can be installed, for example, on a processor of any type.

Said user interface 2 and calculation module 3 comprise software elements, that is to say, software operating instructions which implement the functions claimed/described herein.

The user interface 2 and the calculation module 3 may be implemented in any processor, for example in a remote server.

It should be noted that, advantageously, the computer system 1 allows calculation of the percentage of regeneration additive irrespective of the type thereof; that is to say, unlike what occurs according to the prior art, it is in no way necessary to know the additive in order to derive, based on empirical tables, the optimum value of additive to be used to have a bituminous mix, that is, a final product, with the desired characteristics, in terms of softening temperature or penetration depth at a predetermined temperature (parameters defined by standard EN13108).

According to an aspect, said characteristic size of the bitumen used by the calculation module 3 consists of the softening temperature or the penetration depth at a predetermined temperature, defined in accordance with standard EN13108.

According to another aspect, the calculation module 3 is configured to calculate the percentage of regeneration additive for regenerating the reclaimed bituminous-based material (RAP) on the basis of a second formula which determines a mathematical relationship between the depth of penetration at a predetermined temperature of the reclaimed bituminous-based material (RAP), of the virgin bituminous conglomerate and of the final product.

In short, the first formula relates the depth of penetration to a predetermined temperature of the reclaimed bituminous-based material, the depth of penetration to a predetermined temperature of the virgin bituminous mix and the depth of penetration at a predetermined temperature of the final product.

According to another aspect, said first formula comprises a logarithmic function of the penetration depth at a predetermined temperature of the reclaimed bituminous-based material (RAP), of the virgin bituminous conglomerate and of the final product.

More specifically, according to this aspect, the first formula relates the natural logarithm of the penetration depth at a predetermined temperature of the reclaimed bituminous-based material, the natural logarithm of the penetration depth at a predetermined temperature of the virgin bituminous conglomerate and the natural logarithm of the penetration depth at a predetermined temperature of the final product.

According to another aspect, said first formula is a formula of the type: $a ⋅ lg {pen}_{rejuvenated binder} + b ⋅ lg {pen}_{VIRGIN} = \left(a+b\right) ⋅ lg {pen}_{mix}$ where:
lg *pen_{rejuvenated binder}* is the natural logarithm of the penetration depth at a predetermined temperature of the rejuvenated bitumen, that is to say, the bituminous component of the reclaimed bituminous-based material (RAP) to which the regeneration additive has been applied for regenerating the reclaimed bituminous-based material (RAP).
lg *pen_{VIRGIN}* is the natural logarithm of the depth of penetration at a predetermined temperature of the virgin bitumen;
lg *penₘᵢₓ* is the natural logarithm of the depth of penetration at a predetermined temperature of the finished product obtained from the mix;
   - a and b are two coefficients, the sum of which is equal to 1, and more specifically:
      - a relates to the percentage of bitumen making up the reclaimed bituminous-based material (RAP) on the total bitumen;
      - b relates to the percentage of virgin bitumen on the total bitumen making up the finished product.

It should be noted that the calculation module 3 allows the calculation, based on this formula, of the depth of penetration of the rejuvenated bitumen (*pen_{rejuvenated binder}*).

In fact, the calculation module 3 is configured to determine, using said first formula, the depth of penetration of the rejuvenated bitumen PEN1, and is also configured to calculate, using a further final relationship F1, the percentage of regeneration additive to be added to obtain a reclaimed bituminous-based material whose bitumen has said predetermined value of depth of penetration of the rejuvenated bitumen PEN1 (obtained from the first formula).

The final relationship F1 defines a relationship between:
- the depth of penetration PEN2 of the bitumen of the reclaimed bituminous-based material (that is to say, of the reclaimed bituminous-based material without additive);
- the depth of penetration PEN1 of rejuvenated bitumen;
- the percentage of regeneration additive to be added to obtain a reclaimed bituminous-based material whose bitumen has said predetermined value of depth of penetration of the rejuvenated bitumen PEN1.

The final relationship F1 may consist of a curve, that is, a mathematical function.

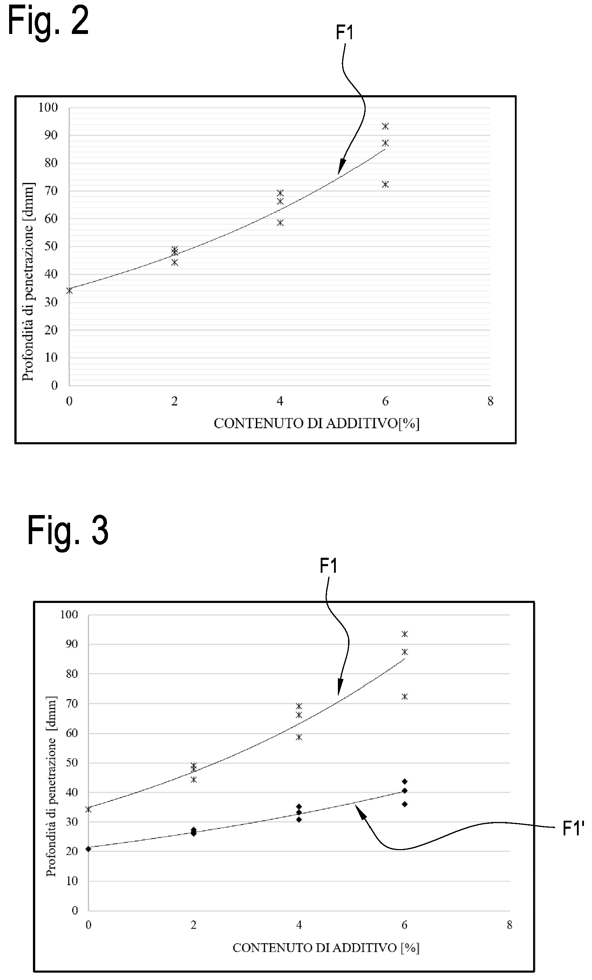

The final relationship F1 (as may be inferred from the graph in Figure 2) is defined, according to an aspect, by an exponential formula, which links the depth of penetration of rejuvenated bitumen PEN1 to the percentage of regeneration additive to be added to obtain a reclaimed bituminous-based material whose bitumen has said predetermined value of depth of penetration of rejuvenated bitumen PEN1.

It should be noted that said value of the depth of penetration PEN2 of the bitumen of the reclaimed bituminous-based material (without the addition of additive) corresponds to the value of the depth of penetration of rejuvenated bitumen determined, on the basis of the final relationship F1, at the zero percentage of regeneration additive.

Figure 2 illustrates, in a graph, the trend of the further final relationship F1 which makes it possible to calculate the percentage of regeneration additive to be added to obtain a reclaimed bituminous-based material whose bitumen has said predetermined value of depth of penetration of the rejuvenated bitumen PEN1.

Obviously, the further final relationship F1 is of the exponential type.

It should be noted that both the first formula and the further final relationship have been obtained experimentally, through a series of tests carried out on additives and reclaimed bituminous-based materials of different types.

In the experimental tests, various types of regeneration additives and reclaimed bituminous-based materials (RAP) of different type were used to derive the above-mentioned final relationship; it has been noted that the percentage of regenerative additive to be added to obtain the desired characteristics of the final bituminous product does not depend on the type of regenerating additive but rather on the characteristics of the reclaimed bituminous-based materials and/or those of virgin bitumen, as clearly expressed by the above formula.

According to another aspect, the user interface 2 is configured to allow the setting of a quality parameter P5 relative to the reclaimed bituminous-based material.

According to this aspect, the calculation module 3 is configured to use as a further final relationship, alternatively, on the basis of said setting of the quality index of the bituminous-based recycled material:
- a first final relationship F1 of the exponential type, having a first exponential coefficient, corresponding to a first setting of the quality parameter;
- a second final relationship F1' of the exponential type, having a second exponential coefficient, corresponding to a second setting of the quality parameter, said first exponential coefficient being greater than the second exponential coefficient.

In short, on the basis of this setting of the quality index of the reclaimed bituminous-based material, the first final relationship F1 or the second final relationship F1' is used, alternatively.

The quality index of reclaimed bituminous-based material expresses, in numerical or binary terms, the distance of the reclaimed bituminous-based material with respect to the virgin bitumen.

It should be noted that if the reclaimed bituminous-based material is close to the virgin bitumen, the second final relationship F1' will be used; vice versa, in the case of reclaimed bituminous-based material distant in terms of quality characteristics (for various reasons, for example ageing, composition of the material etc.) with respect to virgin bitumen, the first final relationship F1 will be used.

Advantageously, therefore, irrespective of the regenerating additive used, according to the system 1 proposed it is possible to determine the percentage of additive to be used extremely quickly and easily, substantially automatically, without having to use experimental type tables. According to another aspect, the calculation module 3 is configured to calculate the percentage of regeneration additive for regenerating the reclaimed bituminous-based material (RAP) on the basis of a second formula which determines a mathematical relationship between the softening temperature of the reclaimed bituminous-based material (RAP), of the virgin bituminous conglomerate and of the final bituminous conglomerate product.

The second formula is an alternative to the first formula.

This softening temperature is the softening temperature at 25°C, defined by standard EN13108.

Said second formula comprises a linear function of the softening temperature at a predetermined temperature of the reclaimed bituminous-based material (RAP), of the virgin bituminous conglomerate and of the final product.

In short, the experimental studies performed by the Applicant have shown that the softening temperature at a predetermined temperature of the reclaimed bituminous-based material (RAP), the softening temperature of the virgin bituminous conglomerate and the softening temperature of the bituminous conglomerate defining the final product are connected to each other by a formula.

More precisely, said second formula is a formula of the type: $\begin{matrix}{T}_{R & B , mix} = a ⋅ {T}_{R & B , rejuvenated binder} + b \\ ⋅ {T}_{R & B , VIRGIN}\end{matrix}$ where:
*T_{R&B,rejuvenated binder}* is the natural logarithm of the softening temperature of the rejuvenated bitumen, that is to say, the bituminous component of the reclaimed bituminous-based material (RAP) to which the regeneration additive has been applied for regenerating the reclaimed bituminous-based material (RAP).
*T_{R&B, VIRGIN}* is the natural logarithm of the softening temperature of the virgin bitumen;
*T_{R&B, mix}* is the natural logarithm of the softening temperature of the finished product obtained from the mix;
   - a and b are two coefficients, the sum of which is equal to 1, and more specifically:
      - a relates to the percentage of bitumen making up the reclaimed bituminous-based material (RAP) on the total bitumen;
      - b relates to the percentage of virgin bitumen on the total bitumen making up the finished product.

According to another aspect, the calculation module 3 is configured for calculating, using the above-mentioned second formula, the softening temperature of the rejuvenated bitumen TEMP1, and is also configured for calculating, using a further final relationship F3, the percentage of regeneration additive to be added to obtain a reclaimed bituminous-based material whose bitumen has said predetermined value of softening temperature of the rejuvenated bitumen TEMP1.

The further final relationship F3 defines a relationship between:
- the softening temperature TEMP2 of the bitumen of the reclaimed bituminous-based material (before applying the additive);
- the softening temperature TEMP1 of the rejuvenated bitumen;
- the percentage of regeneration additive to be added to obtain a reclaimed bituminous-based material whose bitumen has said predetermined value of the softening temperature of the rejuvenated bitumen TEMP1.

According to another aspect, said further final relationship F3 is a linear formula, which associates the softening temperature of the rejuvenated bitumen TEMP1 to the percentage of regeneration additive to be added to obtain a reclaimed bituminous-based material whose bitumen has said predetermined value of the softening temperature of the rejuvenated bitumen TEMP1, said value of the softening temperature TEMP2 of the reclaimed bituminous-based material corresponding to the value of the softening temperature of the rejuvenated bitumen TEMP1 at the zero percentage of regeneration additive.

According to another aspect, the calculation module 3 is configured for calculating the percentage of regeneration additive to be added on the basis of the first and the second formulae and relative final formulae associated with them (as described above), and for comparing said values calculated and making available, as a display, said values calculated using the user interface 2.

According to another aspect, the user interface 2 is configured to allow one or more parameters to be set relative to the regeneration additive.

According to yet another aspect, the user interface 2 is configured to allow the calculation module 3 to be set for calculating the percentage of regeneration additive based on the first formula or the second formula.

Advantageously, in this way, it is possible to use the value considered most reliable for the bituminous macadam being produced, or compare the values to take the final decision on the percentage of additive which will in fact be used for the regeneration.

According to an aspect, the user interface 2 is configured to allow the setting of a maximum value P1 of regeneration additive.

According to an aspect, the user interface 2 is configured to allow the setting of a minimum value P2 of regeneration additive.

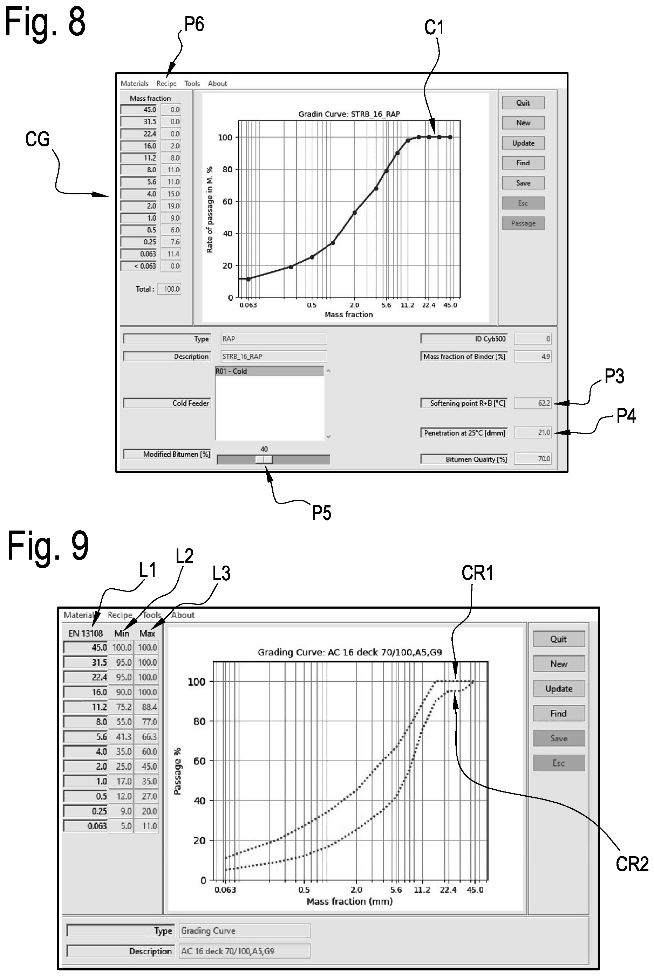

According to another aspect, the user interface 2 is configured for allowing the setting up of grain size curves C1 of the basic materials (see in this regard Figure 8).

According to yet another aspect, the user interface 2 is configured to allow the insertion of the percentage grain size composition in predetermined grain size classes CG of the basic materials (see in this regard Figure 8).

In this regard, Figure 8 clearly illustrates how the interface 2 is configured to display:
- a first list of predetermined values of granular dimensions;
- a second list of lower percentage limit values, corresponding to the values of granular dimensions referred to in the first list.

The curve C1 expresses the grain size of the basic materials; in the X-axis there is in effect the fraction in mass and in the Y-axis the relative percentage.

In this regard, Figure 8 illustrates the user interface 2 configured to allow the selection, that is to say, the setting, of the most interesting parameters of the basic material RAP, that is, of the reclaimed bituminous-based material.

It should be noted that the interface 2, as clearly shown in Figure 8, is configured to allow the softening temperature (parameter P3) and the depth of penetration to be set at a predetermined temperature (parameter P4).

Moreover, the interface 2 is configured to allow the parameter P5 regarding the quality of the reclaimed bituminous-based material (RAP), mentioned above, to be entered.

According to another aspect, as clearly shown in Figure 9, the user interface 2 is configured for displaying;
- a first list L1 of predetermined values of granular dimensions;
- a second list L2 of lower percentage limit values, corresponding to the values of granular dimensions referred to in the first list;
- a third list L3 of higher percentage limit values, corresponding to the values of granular dimensions referred to in the first list.

According to this aspect, the user interface 2 is configured for editing the limit values indicated in the second L2 and third L3 lists.

Figure 9 shows two curves, CR1 and CR2, corresponding, respectively, to the minimum and maximum grain size of the second list L2 and third list L3.

According to another aspect, the user interface 2 is configured to allow the selection of a basic material, in association with a container in which it is contained.

According to another aspect, the user interface 2 is configured to allow the setting, as base material, of a recovery filler, corresponding to a filler recovered from a filter of a plant for producing the bituminous conglomerate.

With regard to the finished product, it should be noted that the user interface 2 is configured to allow a softening temperature of the reclaimed bituminous-based material (RAP) selected relative to the finished product to be set.

Further, the user interface 2 is configured to allow a softening temperature relative to the finished product to be set.

According to another aspect, the user interface 2 is configured to allow a depth of penetration at a predetermined temperature relative to the finished product to be set.

It should be noted that the penetration depth and/or the softening temperature relative to the finished product constitute the calculation parameters used by the calculation module 3 in relation to the first or second formula.

According to yet another aspect, the user interface 2 is configured for:
- selecting a predetermined container (101, 102, 103, 104);
- setting up the grain size composition of the material inside said container (101, 102, 103, 104).

Advantageously, in this way, it is possible to directly select the containers (101, 102, 103, 104) in a mix.

According to another aspect, the user interface 2 is configured to allow the selection of a type of mix.

In effect, there is a menu P6, which allows one or more mixes to be selected.

According to another aspect, the user interface 2 is configured to allow the setting of a maximum percentage P7 of filler which must be present in the final product.

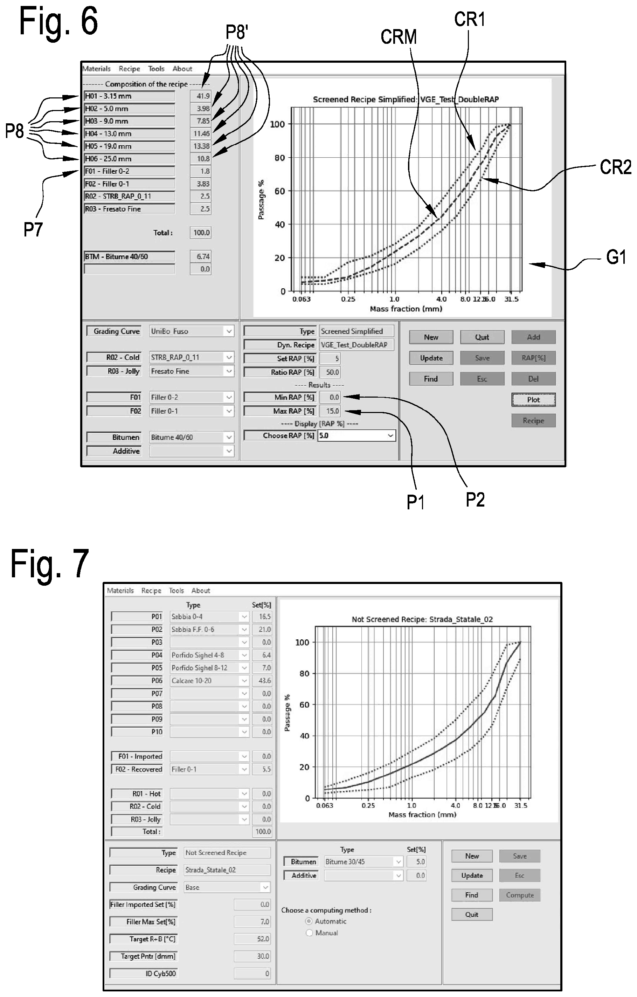

According to another aspect, as clearly illustrated in Figure 6, the computer user interface 2 is configured for selecting one or more aggregate type ingredients and to allow the maximum percentages P8' which must be present in the final product to be set, for one or more basic ingredients of the aggregate type selected P8.

According to another aspect, the user interface 2 is configured for displaying a first predetermined grain size curve CR1 and a second grain size curve CR2, corresponding to lower and upper limit curves of the final product, said curves being represented on a grain size graph G1 having the percentage on the y-axis and the grain size dimension on the x-axis.

According to another aspect, the calculation module 3 is configured for calculating a grain size curve corresponding to the final product, on the basis of the basic ingredients of selected aggregate type P8 and of the respective percentages P8' set and/or calculated, and wherein the user interface 2 is configured for displaying a third curve CRM on said grain size graph G1.

The third curve CRM represents the grain size of the finished product; it must be contained within the first CR1 and second CR2 curve which represent the limit acceptability values.

In fact, according to an aspect, the calculation module 3 is configured for calculating one or more percentages P8' of said basic ingredients of an aggregate type P8 in such a way that said third grain size curve CRM is included, in the grain size graph, in the region between the first curve CR1 and the second curve CR2.

According to yet another aspect, the calculation module 3 is configured for calculating one or more percentages P8' of said basic ingredients of an aggregate type P8 in such a way that said third grain size curve is substantially centred, in the grain size graph G1, in the region between the first and the second curves.

According to another aspect, the user interface 2 is configured to allow the selection of a first and a second type of reclaimed bituminous-based material(RAP), and the setting of:
- a relative ratio between the percentage of reclaimed bituminous-based material (RAP) of the first type and of the second type;
- a maximum percentage value of the reclaimed bituminous-based material (RAP) of the first type and of the second type.

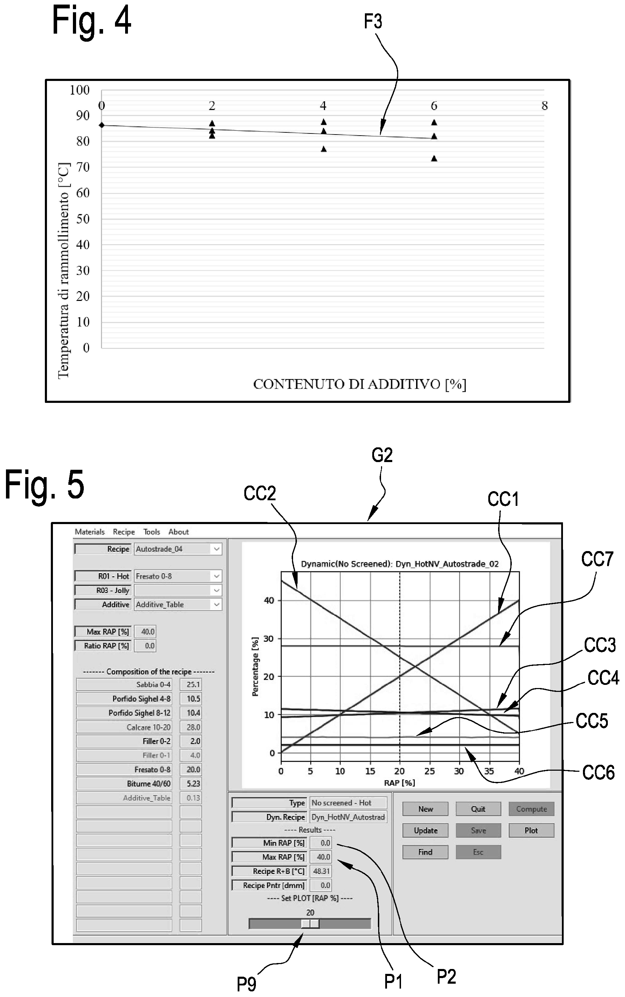

According to yet another aspect, as clearly illustrated in Figure 5, the user interface 2 is configured to display a graph G2 of the materials, comprising in the x-axis the percentage of the reclaimed bituminous-based material (RAP) of the mix and in the y-axis the percentage of each material in the mix for the corresponding value of the reclaimed bituminous-based material (RAP) of the mix.

The calculation module 3 is configured for determining the optimum value of each material in the mix for values of the reclaimed bituminous-based material (RAP) of the mix and for generating, based on said predetermined values, curves (CC1, CC2, CC3, CC4, CC5, CC6, CC7) of the materials.

The user interface 2 is configured for displaying said curves (CC1, CC2, CC3, CC4, CC5, CC6, CC7) on the materials diagram.

According to another aspect, the user interface 2 is configured for displaying the graph G2 of the materials having, on the x-axis, coordinates of between a value 0 and a value corresponding to the maximum percentage value of reclaimed bituminous-based material (RAP) set.

According to another aspect, the user interface 2 is configured for:
- allowing the setting of a percentage of reclaimed bituminous-based material (RAP) and a relative grain size;
- displaying percentages of basic aggregate materials subdivided into grain size classes.

It should be noted that the calculation module 3 is configured for recalculating the percentages of basic aggregate materials subdivided into grain size classes on the basis of a new setting of the percentage of reclaimed bituminous-based material (RAP).

According to another aspect, the user interface 2 is configured to allow a percentage of reclaimed bituminous-based material (RAP) to be set by means of a sliding tool P9.

According to another aspect, the calculation module 3 is configured for recalculating the percentages of basic aggregate materials subdivided into grain size classes on the basis of a new setting of the percentage of reclaimed bituminous-based material (RAP) in a proportional manner.

The invention also defines a plant 100 for producing bituminous macadam starting from basic ingredients, comprising:
- a plurality of dosing containers 101, 102, 103, 104 designed to contain the basic ingredients;
- at least one dryer 106, 107, 108, designed to dry at least a part of the material extracted from said dosing containers 101, 102, 103, 104;
- at least one mixer (109), designed to mix said basic ingredients for making the finished product;
- a computer system 1 according to the appended claims,
- a control unit 105, configured for controlling at least said dosing containers 101, 102, 103, 104, drier 106, 107, 108 and mixer 109.

It should be noted that the control unit 105 is configured to receive information relating to a mix for the production from the computer system 1.

The invention also defines a method for generating mixes for making bituminous macadam from basic ingredients, with said basic ingredients comprising:
- aggregates;
- virgin bitumen;
- a filler, that is to say, a filling product;
- reclaimed bituminous-based material (RAP);
- regeneration additive for regenerating the reclaimed bituminous-based material (RAP).

According to the invention, the method comprises the following steps:
- preparing a computer comprising a user interface (2) and a calculation module 3;
- providing information relating to said mixes using the user interface (2);
- allowing, using the user interface 2, the entering or modification of one or more of said items information relating to the mixes;
- calculating, using the calculation module 3, a percentage of regeneration additive for regenerating the reclaimed bituminous-based material (RAP) on the basis of at least one formula, said formula determining a mathematical relationship between:
- reclaimed bituminous-based material;
- virgin bituminous conglomerate;
- final product obtained from the mix
through a characteristic size of the bitumen.

Advantageously, the method proposed allows the percentage of bitumen to be determined in a particularly simple manner in order to regenerate the reclaimed bituminous-based material, allowing a final product of the bituminous conglomerate type to be obtained having predetermined specifications.

## Claims

1. A computer system (1) for generating mixes for making bituminous macadam starting from basic ingredients, with said basic ingredients comprising:
- aggregates;
- virgin bitumen;
- a filler, that is to say, a filling product;
- reclaimed bituminous-based material (RAP);
- regeneration additive for regenerating the reclaimed bituminous-based material (RAP);
the system (1) being **characterised in that** it comprises, in combination:
- a user interface (2), configured to provide displays of information relating to said mixes and allow the entering or modification of one or more of said items of information relating to the mixes;
- a calculation module (3), configured for calculating a percentage of regeneration additive for regenerating the reclaimed bituminous-based material (RAP) on the basis of at least one formula, said formula determining a mathematical relationship between:
- reclaimed bituminous-based material;
- virgin bituminous conglomerate;
- final product obtained from the mix
through a characteristic size of the bitumen, wherein the calculation module (3) is configured to calculate the percentage of regeneration additive for regenerating the reclaimed bituminous-based material (RAP) on the basis of a second formula which determines a mathematical relationship between the softening temperature of the reclaimed bituminous-based material (RAP), the softening temperature of the virgin bituminous conglomerate and the softening temperature of the final bituminous conglomerate product and wherein said second formula comprises a linear function of the softening temperature at a predetermined temperature of the reclaimed bituminous-based material (RAP), the softening temperature of the virgin bituminous conglomerate and the softening temperature of the final product.

2. The computer system (1) according to the preceding claim, wherein said characteristic size of the bitumen consists of the softening temperature or the penetration depth at a predetermined temperature, defined in accordance with standard EN13108.

3. The computer system (1) according to the preceding claim, wherein the calculation module (3) is configured for calculating the percentage of regeneration additive for regenerating the reclaimed bituminous-based material (RAP) on the basis of a first formula which determines a mathematical relationship between the depth of penetration at a predetermined temperature of the reclaimed bituminous-based material (RAP), the depth of penetration at a predetermined temperature of the virgin bituminous conglomerate and the depth of penetration at a predetermined temperature of the final product.

4. The computer system (1) according to the preceding claim, wherein said first formula comprises a logarithmic function of the depth of penetration at a predetermined temperature of the reclaimed bituminous based material (RAP), the depth of penetration at a predetermined temperature of the virgin bituminous conglomerate and the depth of penetration at a predetermined temperature of the final product.

5. The computer system (1) according to the preceding claim, wherein said first formula is a formula of the type: $a ⋅ lg {pen}_{rejuvenated binder} + b ⋅ lg {pen}_{VIRGIN} = \left(a+b\right) ⋅ lg {pen}_{mix}$ where:
lg *pen_{rejuvenated binder}* is the natural logarithm of the penetration depth at a predetermined temperature of the rejuvenated bitumen, that is to say, the bituminous component of the reclaimed bituminous-based material (RAP) to which the regeneration additive has been applied for regenerating the reclaimed bituminous-based material (RAP);
lg *pen_{VIRGIN}* is the natural logarithm of the depth of penetration at a predetermined temperature of the virgin bitumen;
lg *penₘᵢₓ* is the natural logarithm of the depth of penetration at a predetermined temperature of the finished product obtained from the mix;
- a and b are two coefficients, the sum of which is equal to 1, and more specifically:
- a relates to the percentage of bitumen making up the reclaimed bituminous-based material (RAP) on the total bitumen;
- b relates to the percentage of virgin bitumen on the total bitumen making up the finished product.

6. The computer system (1) according to the preceding claim, wherein the calculation module (3) is configured to determine, using said first formula, the depth of penetration of the rejuvenated bitumen (PEN1), and is also configured to calculate, using a further final relationship (F1, F1'), the percentage of regeneration additive to be added to obtain a reclaimed bituminous-based material whose bitumen has said value determined using the first formula for depth of penetration of the rejuvenated bitumen (PEN1), said final relationship (F1, F1') defining a relationship between:
- the depth of penetration (PEN2) of the bitumen of the reclaimed bituminous-based material;
- the depth of penetration (PEN1) of the rejuvenated bitumen, that is to say, of the bitumen of the reclaimed bituminous-based material (RAP) to which the regeneration additive has been applied;
- the percentage of regeneration additive to be added to obtain a reclaimed bituminous-based material whose bitumen has said predetermined value of depth of penetration of the rejuvenated bitumen (PEN1).

7. The computer system (1) according to the preceding claim, wherein said final relationship (F1, F1') is defined by an exponential function, which links the depth of penetration of the rejuvenated bitumen (PEN1) to the percentage of regeneration additive to be added to obtain a reclaimed bituminous-based material whose bitumen has said predetermined value of depth of penetration of the rejuvenated bituminous (PEN1), said depth of penetration value (PEN2) of the bitumen of the reclaimed bituminous-based material corresponding to the value of the depth of penetration of the rejuvenated bitumen (PEN1) at the zero percentage of regeneration additive.

8. The computer system (1) according to the preceding claim, wherein the user interface (2) is configured to allow the setting of a quality parameter relative to the reclaimed bituminous-based material, and wherein the calculation module (3) is configured for using as final relationship (F1, F1'), alternatively, on the basis of said setting of the quality index of the reclaimed bituminous-based material:
- a first final relationship (F1) of the exponential type, having a first exponential coefficient, corresponding to a first setting of the quality parameter;
- a second final relationship (F1') of the exponential type, having a second exponential coefficient, corresponding to a second setting of the quality parameter, said first exponential coefficient being greater than the second exponential coefficient.

9. The computer system (1) according to any one of the preceding claims, wherein said second formula is a formula of the type: $\begin{matrix}{T}_{R & B , mix} = a ⋅ {T}_{R & B , rejuvenated binder} + b \\ ⋅ {T}_{R & B , VIRGIN}\end{matrix}$ Where:
*T_{R&B,rejuvenated binder}* is the natural logarithm of the softening temperature of the rejuvenated bitumen, that is to say, the bituminous component of the reclaimed bituminous-based material (RAP) to which the regeneration additive has been applied for regenerating the reclaimed bituminous-based material (RAP).
*T_{R&B, VIRGIN}* is the natural logarithm of the softening temperature of the virgin bitumen;
*T_{R&B, mix}* is the natural logarithm of the softening temperature of the finished product obtained from the mix;
- a and b are two coefficients, the sum of which is equal to 1, and more specifically:
- a relates to the percentage of bitumen making up the reclaimed bituminous-based material (RAP) on the total bitumen;
- b relates to the percentage of virgin bitumen on the total bitumen making up the finished product;

10. The computer system (1) according to the preceding claim, wherein the calculation module (3) is configured for calculating, using the second formula, the softening temperature of the rejuvenated bitumen (TEMP1), and is also configured for calculating, using a further final relationship (F3), the percentage of regeneration additive to be added to obtain a reclaimed bituminous-based material whose bitumen has said predetermined value of softening temperature of the rejuvenated bitumen (TEMP1), said final relationship (F3) comprising a relationship between:
- the softening temperature (TEMP2) of the bitumen of the reclaimed bituminous-based material;
- the softening temperature (TEMP1) of the bitumen of the rejuvenated reclaimed bituminous-based material to which the additive has been applied;
- the percentage of regeneration additive to be added to obtain a reclaimed bituminous-based material whose bitumen has said predetermined value of the softening temperature of the rejuvenated bitumen (TEMP1).

11. The computer system (1) according to the preceding claim, wherein the final relationship (F3) is a linear function, which links the softening temperature of the rejuvenated bitumen (PEN1) to the percentage of regeneration additive to be added to obtain a reclaimed bituminous-based material whose bitumen has said predetermined value of the softening temperature of the rejuvenated bitumen (PEN1), said value of the softening temperature (PEN2) of the reclaimed bituminous-based material corresponding to the value of the softening temperature of the rejuvenated bitumen (PEN1) at the zero percentage of regeneration additive.

12. The computer system (1) according to any one of the preceding claims and claim 4, wherein the calculation module (3) is configured to calculate the percentage of regeneration additive to be added on the basis of the first and second formula, and to compare said calculated values of percentage of regeneration additive and to make available through the user interface (2), as a display, said calculated values of percentage of regeneration additive.

13. A method for generating mixes for making bituminous macadam from basic ingredients, with said basic ingredients comprising:
- aggregates;
- virgin bitumen;
- a filler, that is to say, a filling product;
- reclaimed bituminous-based material (RAP);
- regeneration additive for regenerating the reclaimed bituminous-based material (RAP);
the method including the following steps:
- preparing a computer comprising a user interface (2) and a calculation module (3);
- providing information relating to said mixes using the user interface 2;
- allowing, using the user interface (2), the entering or modification of one or more of said items information relating to the mixes;
- calculating, using the calculation module (3), a percentage of regeneration additive for regenerating the reclaimed bituminous-based material (RAP) on the basis of at least one formula, said formula determining a mathematical relationship between:
- reclaimed bituminous-based material;
- virgin bituminous conglomerate;
- final product obtained from the mix
through a characteristic size of the bitumen, wherein the calculating phase comprises a phase of calculating the percentage of regeneration additive for regenerating the reclaimed bituminous-based material (RAP) on the basis of a second formula which determines a mathematical relationship between the softening temperature of the reclaimed bituminous-based material (RAP), the softening temperature of the virgin bituminous conglomerate and the softening temperature of the final bituminous conglomerate product and wherein said second formula comprises a linear function of the softening temperature at a predetermined temperature of the reclaimed bituminous-based material (RAP), the softening temperature of the virgin bituminous conglomerate and the softening temperature of the final product.
